# EUROPEAN PATENT SPECIFICATION

(11) **EP 1 851 076 B1**
(45) Date of publication and mention of the grant of the patent: **03.12.2008**
(21) Application number: 06710843.1
(22) Date of filing: 07.02.2006
(51) Int. Cl.: B60H 3/00

(54) **VEHICLE ACCESSORIES FOR ENHANCING SMELLS**
FAHRZEUGZUBEHÖR ZUR VERBESSERUNG VON GERÜCHEN
ACCESSOIRES DE VEHICULE

(30) Priority: 14.02.2005 EP 05101050
(43) Date of publication of application: 07.11.2007
(73) Proprietor: Koninklijke Philips Electronics N.V., 5621 BA Eindhoven (NL)
(72) Inventor: HOLLEMANS, Gerrit, NL-5656 AA Eindhoven (NL); SEMPEL, Adrianus, NL-5656 AA Eindhoven (NL); VAN LOENEN, Evert, J., NL-5656 AA Eindhoven (NL)
(74) Representative: Schouten, Marcus Maria
(86) International application number: PCT/IB2006/050391
(87) International publication number: WO 2006/085264

(56) References cited:
- EP-A- 1 354 738
- EP-A- 1 439 083
- DE-B3- 10 301 214
- US-A1- 2004 072 533
- PATENT ABSTRACTS OF JAPAN vol. 014, no. 212 (M-0969), 2 May 1990 (1990-05-02) & JP 02 048217 A (NIPPON DENSO CO LTD), 19 February 1990 (1990-02-19)
- PATENT ABSTRACTS OF JAPAN vol. 2003, no. 02, 5 February 2003 (2003-02-05) & JP 2002 283844 A (DENSO CORP), 3 October 2002 (2002-10-03)

## Description

### FIELD OF THE INVENTION

The present invention relates to vehicle accessories, for example to accessories operable to enhance travelling experience, safety and comfort for vehicle passengers and their drivers. Moreover, the invention also relates to methods of enhancing travelling experience using such accessories.

### BACKGROUND TO THE INVENTION

Contemporary road vehicles include automobiles, cars, vans and trucks amongst others. These contemporary vehicles are increasingly provided with safety features and user comforts to an extent that passengers and drivers feel cocooned in such vehicles and thereby isolated from external surroundings. Whereas such isolation can increase passenger and driver comfort, it can also increase a risk of accident because drivers and passengers often become unaware of their speed of travel and external events occurring around them. Moreover, whereas travelling in an open-top sports car provides drivers and passengers with a sensation of speed and enables them to sense fully their external environment, for example smells, not all passengers and drivers, especially in adverse weather conditions such as rainy weather, are enthusiastic to travel in open-topped sports cars.

It is known, for example as described in a published Japanese patent application no. JP-2002283844, to include smell generators within road vehicles. These smell generators are conveniently implemented in co-operation with air-conditioning systems included in the vehicles. The aforesaid patent application is concerned with a technical problem of preventing a newly emitted smell from mixing with a previously emitted smell. Such mixing is at least partially avoided by employing a fresh-air mode of operation to purge an interior region of a road vehicle in which passengers and the driver are accommodated to remove the previously emitted smell before introducing the newly emitted smell. The fresh-air mode involves employing maximum wind force for enhanced purging rate before returning to a lower force for circulating the newly introduced smell. The newly emitted small can, for example, correspond to the smell of flowers. By introducing these smells, driver awareness of external conditions can be enhanced and thereby driver and passenger experience can be enhanced and safety can be improved by increased awareness in response to the generated smells.

It is further known from JP-02048217, which is considered as the closest prior art, to release one fragrance in the interior of the vehicle when specific conditions are detected by sensors inside and outside the vehicle.

The inventors have appreciated that contemporary in-car smell generators are limited in their repertoire of smells and often are unreliable when required to detect relevant external smells. Such limited repertoire and unreliability represent a technical problem, which the present invention seeks to address.

### SUMMARY OF THE INVENTION

It is an object of the invention to provide a vehicle accessory operable to enhance vehicle passenger and driver safety and experience, for example in regard of smell recognition and smell generation.

According to a first aspect of the present invention, there is provided a vehicle accessory for enhancing smells within a vehicle, the accessory comprising:
(a) sensors for sensing at least one of visual objects and smells present in an environment surrounding the vehicle and generating corresponding sensor signals;
(b) a processor for receiving the sensor signals and analysing them for detecting the presence of one or more sensed objects and/or one or more sensed smells in the environment, the processor being operable to filter a subset of the one or more sensed objects and/or one or more sensed smells; and
(c) an odour source coupled to the processor for receiving instructions there from to generate and release into the vehicle one or more smells corresponding to the subset of the one or more sensed objects and/or one or more sensed smells.

The invention is of advantage in that it is capable of enhancing vehicle passenger and driver safety and experience.

Optionally, in the accessory, the processor is operable to employ a neural network algorithm, a template matching algorithm and/or a correlation algorithm for analysing the sensor signals for detecting the presence of the one or more sensed objects and/or the one or more sensed smells. Such neural network algorithms, templates matching algorithms and correlation algorithms are effective at identifying similarity between objects represented as data.

Optionally, in the accessory, the odour source is operable to function in conjunction with an air-recirculating system or air-conditioning system included in the vehicle for purging smells from an interior region of the vehicle which are no longer required by the processor, and for re-circulating required smells within the interior region of the vehicle for reducing a consumption of odour generating materials in the odour source. Such an implementation of the accessory is susceptible to rendering it more economical in its operative use of smell-generating materials.

Optionally, in the accessory, the sensors for sensing smells present in the environment are implemented as an array of conductive polymer sensors. Such sensors are suitable to use on account of their low cost, small size, robustness and low power consumption.

More optionally, in the accessory, the sensors for sensing smells present in the environment are capable of being heated to an elevated temperature to at least partially expel accumulated contamination thereon. On account of being able to expel contamination, the polymer sensors are especially appropriate to employ in view of the amount of air-borne pollution encountered in road and motorway environments.

More optionally, in the accessory, the sensors for sensing smells present in the environment are provided with one or more sources of a calibration gas for use in periodically calibrating the sensors. Provision of such calibration is of value in determining when the sensors are no longer correctly functioning and are therefore in need of replacement as well as enabling the processor to adapt to changes in sensor sensitivity when executing its analysis algorithms. Reliability of the accessory is thereby potentially enhanced.

Optionally, in the accessory, the odour source is provided with a plurality of materials, which are susceptible to being vaporised by thermal, and/or ultrasonic excitation to generate smells for release into the vehicle. Thermal and ultrasonic evaporation techniques are especially suitable for use in the vehicle. For example, ultrasonic evaporation is potentially very responsive and fast.

Preferably, in the accessory, the plurality of materials susceptible to generating odours for releasing into the vehicle is provided as a replaceable cartridge. Such a cartridge renders the accessory easier to service in use to ensure that it continues to function reliably.

Optionally, in the accessory, the sensors for sensing visual objects present in the environment are implemented as one or more cameras directed in at least one of: in a forward direction of travel of the vehicle, in a lateral direction relative to the direction of travel of the vehicle. More optionally, the sensors for sensing visual objects present in the environment are mounted in one or more positions within the vehicle whereat their field of vision is periodically subject to a cleaning operation to remove vision-occluding debris. Beneficially, windscreen wipers can be synergistically employed for such cleaning purposes.

According to a second aspect of the invention, there is provided a method of enhancing smells within a vehicle using a vehicle accessory, the method comprising steps of:
(a) sensing using sensors at least one of visual objects and smells present in an environment surrounding the vehicle and generating corresponding sensor signals;
(b) receiving the sensor signals at a processor and analysing them therein for detecting the presence of one or more sensed objects and/or one or more sensed smells in the environment, and filtering in the processor a subset of the one or more sensed objects and/or one or more sensed smells; and
(c) receiving instructions at an odour source coupled to the processor to generate and release into the vehicle one or more smells corresponding to the subset of the one or more sensed objects and/or one or more sensed smells.

It will be appreciated that features of the invention are susceptible to being combined in any combination without departing from the scope of the invention.

### DESCRIPTION OF THE DIAGRAMS

Embodiments of the invention will now be described, by way of example only, with reference to the following diagrams wherein:
Figure 1 is a schematic illustration of a road vehicle travelling within an environment, the vehicle including an accessory implemented as an odour generator; and
Figure 2 is a schematic illustration of a replaceable smell-generating cartridge of the odour generator of Figure 1.

### DESCRIPTION OF EMBODIMENTS OF THE INVENTION

An overview of the present invention will firstly be described, followed by a more detailed description of example embodiments of the invention. In a road vehicle, a user inside the vehicle is at least partially isolated from smells existing outside the vehicle. Opening a window of the vehicle is not especially desirable on account of wind noise, ingress of precipitation into the vehicle and heat input to the vehicle, which can conflict, with the provision of air-conditioning within the vehicle. A further issue is that some sources of smell, for example flowers in a field or farms, are often at too great a distance from the vehicle to give rise to a sufficiently detectable smell to the user within the vehicle; the user thereby does not enjoy the benefit of sensing desirable smells which would otherwise enhance user-experience and enjoyment, as well as improving user awareness and concentration.

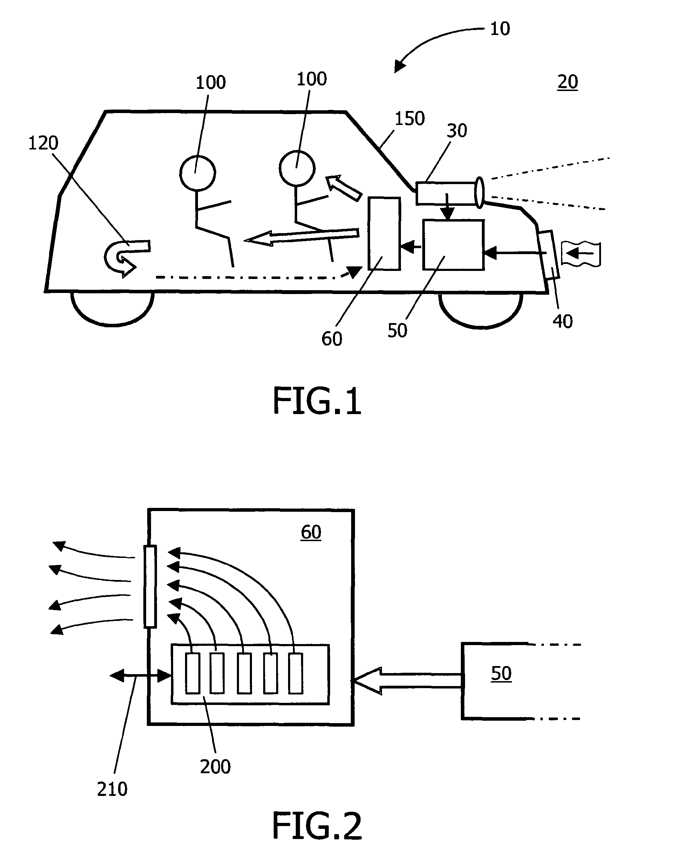

In order to at least partially address such problems experienced by the user, the present invention concerns an odour generator. In Figure 1, there is shown a road vehicle indicated generally by 10 travelling within an external environment 20. The road vehicle 10 includes the odour generator which comprises an optical image sensor 30, one or more smell sensors 40, a processor 50 for performing smell analysis and image analysis of signals generated by the image sensor 30 and the one or more smell sensors 40 as well as executing decisions concerning likely smells or potential smell sources present, and an odour source 60 for directing generated odours to one or more users 100, for example a driver and optionally one or more passengers, included within the vehicle 10. The optical image sensor 30 is optionally implemented as a camera comprising an array of photosensitive elements, for example a contemporary charge coupled device (CCD) or complementary metal oxide semiconductor (CMOS) pixel camera, thereby being operable to provide the processor 50 with images of features and objects present in the external environment 20. More optionally, the optical sensor 30 is orientated to sense a forward field of view experienced by a driver of the vehicle 10 when the vehicle 10 is travelling in a forward direction. Yet more optionally, the optical sensor 30 comprises a plurality of cameras directed not only in the forward direction but also laterally in relation to the vehicle 10.

In operation, the optical image sensor 30 and the one or more smell sensors 40 provide signals to the processor 50. The processor 50 is operable to analyse the signals received thereat to determine one or more of the following:
(a) a presence of smells in the environment 20 without a corresponding visually sensed object being identified;
(b) a presence of visually-sensed objects which are capable of giving rise to smells but which are not represented in the signal from the one or more smell sensors 40; and
(c) a presence of smells in the environment 20 which are confirmed by images of corresponding smell generating objects in the signal from the optical sensor 30.

In cases (a) and (b), the processor 50 beneficially requires there to be an unambiguous indication of a given smell or smell generating object present in the environment 20 before progressing to instruct the odour source 60 to generate a corresponding smell within the vehicle 10 depending on the nature of the smell or smell source identified. The processor 50 is thereby capable of functioning as a smell filter on account of determining:
(i) whether or not the odour source 60 has a type of smell corresponding to the identified smell or smell generating object;
(ii) whether or not the identified smell or smell generating object's smell is a type of smell which is desirable, for example in response to user's preferred smell profile settings input to the processor 50, to have circulating within the vehicle 10. In this respect, the processor 50 includes an object classifier and is also provided with a library of categories of objects and their associated status, namely whether or not smells corresponding to the objects are to be mimicked within the vehicle 10 or not.

The processor 50 optionally employs a form of neural-network recognition method, which is adaptable to learn to identify new types of smells and smell-generating objects in the environment 20. Moreover, the processor 50 is also operable to check for the presence of both a smell and visual confirmation thereof in the environment 20.
Furthermore, the processor 50 is optionally also operable to control operation of the odour source 60 to purge an earlier smell circulating within the vehicle 10 before introducing a subsequent later smell in response to the signal received at the processor 50 from the sensors 30, 40. The odour source 60 is operable to emit streams of air, which have been enhanced with one, or more smells in response to the odour source 60 receiving instructions from the processor 50. In order to limit the amount of material consumed in the odour source 60 in generating smells within the vehicle 10, air within the vehicle 10 is preferably re-circulated as denoted by an arrow 120.

Implementations of the optical sensor 30, the smell sensor 40, the processor 50 and the odour source 60 will now be elucidated in greater detail.

The odour source 60 can be implemented in several alternative ways. Smell-generating liquid, gel or organic polymer are beneficially vaporised in the odour source 60 by ultrasonic vaporisation, thermal evaporation or nebulization to generate smells in the vehicle in response to instructions received at the odour source 60 from the processor 50. Ultrasonic generation of smells in the vehicle 10 is preferred to thermal evaporation generation of smells on account of more advantageous rapidity of response and also on account of lower energy consumption.

The one or more smell sensors 40 are beneficially mounted behind an engine grill of the vehicle 10 or in a front bumper of the vehicle 10. Moreover, the optical sensor 30 can be mounted behind a windscreen 150 of the vehicle 10; such a mounting arrangement is of advantage in that windscreen wipers of the vehicle 10 are also capable of maintaining a clear field of view for the optical sensor 30.

The processor 50 is programmed to recognize images of smell-generating objects in the environment 20, for example flowers, cows, factories and such like by conventional image analysis techniques. Such conventional techniques include one or more of: visual template matching, image correlation, and a neural network approach.

The processor 50 is beneficially operable to employ a naive Bayesian classifier, which is fed with signals corresponding to pleasant and unpleasant smells for appropriately programming the processor 50.

The one or more smell sensors 40 are beneficially implemented using arrays of conducting polymer composite vapor detectors. These detectors employ arrays of chemically sensitive resistors fabricated from composites of carbon black particles with insulating organic polymers. Such detectors can be fabricated to very small size, exhibit low power consumption in use and are of low weight. The organic polymers optionally comprise one or more of: poly(ethylene oxide), poly(55% ethylene-co-45% vinyl acetate), poly(72% butadiene-co-28% styrene), poly(vinyl carbazole), poly(vinyl acetate), poly(caprolactone), polysulfone, poly(vinyl pyrrolidone), poly(4-vinyl phenol), poly (methyloctadecyl siloxane). By employing an array of such detectors, said detectors exhibiting mutually different sensitivities to various types of smell, a neural-network analysis applied to process conductivity measures of the detectors can enable the detection of several different types of smell present in the environment 20. If required, offsets and instabilities in the detectors can be corrected for by periodically purging the detectors with a known test gas sample. The detectors are optionally fabricated either onto ceramic or silicon substrates. The detectors are operable to detect odours and smells by virtue of polymers employed in the detectors absorbing vapor and in response spatially swelling up, thereby altering mutual spatial separation of the carbon black particles and thus modulating electrical conductivity of the detectors.

Alternatively, or additionally, the one or more smell sensors 40 can be implemented so as to be operable at an elevated temperature for automatically burning-off any contamination which accumulates on the one or more sensors 40.

The aforementioned neural network programmable to recognise specific types of objects and specific types of smells in the environment 20 is optionally implemented as software executable on the processor 50.

The odour source 60 is optionally implemented so that various liquids, gels and polymers capable of giving rise to smells within the vehicle 10 are provided in the form of a replaceable cartridge 200 illustrated in Figure 2 which the user 100 can periodically replace as required as denoted by 210, for example at each servicing session of the vehicle 10, for example for every 100 hours or 10000 km of distance travelled by the user 100.

The processor 50 is also beneficially provided with one or more of: an input-output communication port and a wireless data link so that the processor 50 can be periodically updated with more advanced object recognition software and smell recognition software so that the processor 50 can be updated during the operating lifetime of the vehicle 10.

An air-conditioning system (not shown in Figure 1) included as a part of the vehicle 10 for controlling an inner climate of the vehicle 10 where the users 100 sit is preferably arranged to function in co-operation with the odour source 60. For example, when the processor 50 identifies in operation that an earlier smell introduced into the vehicle 10 is to be eliminated there from, for example in response to a corresponding object disappearing from view of the optical sensor 30 or a corresponding external smell no longer being detected by the one or more smell sensors 40, the processor 50 can be arranged to instruct the air-conditioning system to ventilate the interior of the vehicle 10 to purge the earlier smell away.

The sensors 30, 40, the processor 50 and the odour source 60 are optionally designed to be a system, which can be retrofitted to existing road vehicles as an upgrade. Optionally, the processor 50 is implemented using pre-existing computing hardware included in the road vehicle.

Although smell generation in response to visual input and smell input in the context of road vehicles is described in the foregoing, the present invention has potentially other uses, for example in buildings, in hotels, in restaurants, in trains, in aeroplanes, on boats and ships to mention a few examples.

In the accompanying claims, numerals and other symbols included within brackets are included to assist understanding of the claims and are not intended to limit the scope of the claims in any way.

It will be appreciated that embodiments of the invention described in the foregoing are susceptible to being modified without departing from the scope of the invention as defined by the accompanying claims.

Expressions such as "comprise", "include", "incorporate", "contain", "is" and "have" are to be construed in a non-exclusive manner when interpreting the description and its associated claims, namely construed to allow for other items or components which are not explicitly defined also to be present. Reference to the singular is also to be construed to be a reference to the plural and vice versa.

## Claims

1. A vehicle accessory (30, 40, 50, 60) for enhancing smells within a vehicle (10), the accessory (30, 40, 50, 60) comprising:
(a) sensors (30, 40) for sensing at least one of visual objects and smells present in an environment (20) surrounding the vehicle (10) and generating corresponding sensor signals;
(b) a processor (50) for receiving the sensor signals and analysing them for detecting the presence of one or more sensed objects and/or one or more sensed smells in the environment (20), the processor (50) being operable to filter a subset of the one or more sensed objects and/or one or more sensed smells; and
(c) an odour source (60) coupled to the processor (50) for receiving instructions there from to generate and release into the vehicle (10) one or more smells corresponding to the subset of the one or more sensed objects and/or one or more sensed smells.

2. A vehicle accessory (30, 40, 50, 60) as claimed in Claim 1, wherein the processor (50) is operable to employ a neural network algorithm, a template matching algorithm and/or a correlation algorithm for analysing the sensor signals for detecting the presence of the one or more sensed objects and /or the one or more sensed smells.

3. A vehicle accessory (30, 40, 50, 60) as claimed in Claim 1, wherein the odour source (60) is operable to function in conjunction with an air-recirculating system or air-conditioning system included in the vehicle (10) for purging smells from an interior region of the vehicle (10) which are no longer required by the processor (50), and for re-circulating required smells within the interior region of the vehicle (10) for reducing a consumption of odour generating materials (200, 210) in the odour source (60).

4. A vehicle accessory (30, 40, 50, 60) as claimed in Claim 1, wherein the sensors (40) for sensing smells present in the environment (20) are implemented as an array of conductive polymer sensors.

5. A vehicle accessory (30, 40, 50, 60) as claimed in Claim 4, wherein the sensors (40) for sensing smells present in the environment (20) are capable ofbeing heated to an elevated temperature to at least partially expel accumulated contamination thereon.

6. A vehicle accessory (30, 40, 50, 60) as claimed in Claim 4, wherein the sensors (40) for sensing smells present in the environment (20) are provided with one or more sources of a calibration gas for use in periodically calibrating the sensors (40).

7. A vehicle accessory (30, 40, 50, 60) as claimed in Claim 1, wherein the odour source (60) is provided with a plurality of materials which are susceptible to being vaporised by thermal and/or ultrasonic excitation to generate smells for release into the vehicle (10).

8. A vehicle accessory (30, 40, 50, 60) as claimed in Claim 7, wherein the plurality of materials susceptible to generating odours for releasing into the vehicle (10) is provided as a replaceable cartridge (200).

9. A vehicle accessory (30, 40, 50, 60) as claimed in Claim 1, wherein the sensors (30) for sensing visual objects present in the environment (20) are implemented as one or more cameras (30) directed in at least one of: in a forward direction of travel of the vehicle (10), in a lateral direction relative to the direction of travel of the vehicle (10).

10. A vehicle accessory (30, 40, 50, 60) as claim in Claim 9, wherein the sensors (30) for sensing visual objects present in the environment (20) are mounted in one or more positions within the vehicle (10) whereat their field of vision is periodically subject to a cleaning operation to remove vision-occluding debris.

## Patentansprüche

1. Fahrzeugzubehör (30, 40, 50, 60) zur Verbesserung von Gerüchen innerhalb eines Fahrzeugs (10), wobei das Zubehör (30, 40, 50, 60) umfasst:
(a) Sensoren (30, 40) zum Abtasten von mindestens einem visuellen Objekt und in einer das Fahrzeug (10) umgebenden Umgebung (20) vorhandenen Gerüchen sowie Erzeugen entsprechender Sensorsignale,
(b) einen Prozessor (50) zum Empfang der Sensorsignale und Analysieren derselben, um die Präsenz eines oder mehrerer abgetasteten Objekte und/oder eines oder mehrerer abgetasteten Gerüche in der Umgebung (20) zu detektieren, wobei der Prozessor (50) so betreibbar ist, dass er eine Teilmenge des einen oder der mehreren abgetasteten Objekte und/oder des einen oder der mehreren abgetasteten Gerüche filtert, sowie
(c) eine Geruchsquelle (60), die an den Prozessor (50) gekoppelt ist, um von diesem Anweisungen zu empfangen, um einen oder mehrere Gerüche entsprechend der Teilmenge des einen oder der mehreren abgetasteten Objekte und/oder des einen oder der mehreren abgetasteten Gerüche zu erzeugen und in das Fahrzeug (10) freizusetzen.

2. Fahrzeugzubehör (30, 40, 50, 60) nach Anspruch 1, wobei der Prozessor (50) so betreibbar ist, dass er zum Analysieren der Sensorsignale einen neuronalen Netzwerkalgorithmus, einen Template-Matching-Algorithmus und/oder einen Korrelationsalgorithmus anwendet, um die Präsenz des einen oder der mehreren Objekte und/oder des einen oder der mehreren Gerüche zu detektieren.

3. Fahrzeugzubehör (30, 40, 50, 60) nach Anspruch 1, wobei die Geruchsquelle (60) so betreibbar ist, dass sie in Verbindung mit einem in dem Fahrzeug (10) enthaltenen Luftumwälzsystem oder einer Klimaanlage arbeitet, um Gerüche, die von dem Prozessor (50) nicht mehr benötigt werden, aus einem Innenbereich des Fahrzeugs (10) zu entfernen und benötigte Gerüche in dem Innenbereich des Fahrzeugs (10) umzuwälzen, um einen Verbrauch von Geruchserzeugungsmaterialien (200, 210) in der Geruchsquelle (60) zu reduzieren.

4. Fahrzeugzubehör (30, 40, 50, 60) nach Anspruch 1, wobei die Sensoren (40) zum Abtasten von in der Umgebung (20) vorhandenen Gerüchen als ein Array von leitenden Polymersensoren implementiert werden.

5. Fahrzeugzubehör (30, 40, 50, 60) nach Anspruch 4, wobei die Sensoren (40) zum Abtasten von in der Umgebung (20) vorhandenen Gerüchen bis zu einer erhöhten Temperatur erwärmt werden können, um sich darauf angesammelte Verunreinigung zumindest teilweise zu entfernen.

6. Fahrzeugzubehör (30, 40, 50, 60) nach Anspruch 4, wobei die Sensoren (40) zum Abtasten von in der Umgebung (20) vorhandenen Gerüchen mit einer oder mehreren Quellen eines Kalibrierungsgases versehen sind, welches bei der periodischen Kalibrierung der Sensoren (40) verwendet wird.

7. Fahrzeugzubehör (30, 40, 50, 60) nach Anspruch 1, wobei die Geruchsquelle (60) mit einer Vielzahl von Materialien versehen ist, die dazu geeignet sind, durch thermische und/oder Ultraschallanregung verdampft zu werden, um Gerüche zur Freisetzung in das Fahrzeug (10) zu erzeugen.

8. Fahrzeugzubehör (30, 40, 50, 60) nach Anspruch 7, wobei die Vielzahl von Materialien, die dazu geeignet sind, Gerüche zur Freisetzung in das Fahrzeug (10) zu erzeugen, als auswechselbare Patrone (200) vorgesehen ist.

9. Fahrzeugzubehör (30, 40, 50, 60) nach Anspruch 1, wobei die Sensoren (30) zur Abtastung von in der Umgebung (20) vorhandenen, visuellen Objekten als eine oder mehrere Kameras (30) implementiert werden, die zumindest in eine Vorwärtsfahrtrichtung des Fahrzeugs oder in eine seitliche Richtung relativ zu der Fahrtrichtung des Fahrzeugs (10) gerichtet sind.

10. Fahrzeugzubehör (30, 40, 50, 60) nach Anspruch 9, wobei die Sensoren (30) zur Abtastung von in der Umgebung (20) vorhandenen, visuellen Objekten in einer oder mehreren Positionen innerhalb des Fahrzeugs (10) angebracht werden, wobei deren Sehfeld periodisch einem Reinigungsverfahren unterworfen wird, um die Sicht versperrende Fremdkörper zu entfernen.

## Revendications

1. Accessoire de véhicule (30, 40, 50, 60) pour améliorer les odeurs à l'intérieur d'un véhicule (10), l'accessoire (30, 40, 50, 60) comprenant :
(a) des capteurs (30, 40) pour détecter au moins l'un des objets visuels et des odeurs présents dans un environnement (20) entourant le véhicule (10) et générer des signaux de capteurs correspondants ;
(b) un processeur (50) pour recevoir les signaux de capteurs et les analyser pour détecter la présence d'un ou plusieurs objets détectés et/ou d'une ou plusieurs odeurs détectées dans l'environnement (20), le processeur (50) étant utilisable pour filtrer un sous-ensemble de l'un ou plusieurs objets détectés et/ou de l'une ou plusieurs odeurs détectées ; et
(c) une source d'odeurs (60) couplée au processeur (50) pour recevoir des instructions de celui-ci pour générer et dégager dans le véhicule (10) une ou plusieurs odeurs correspondant au sous-ensemble de l'un ou plusieurs objets détectés et/ou de l'une ou plusieurs odeurs détectées.

2. Accessoire de véhicule (30, 40, 50, 60) selon la revendication 1, dans lequel le processeur (50) est utilisable pour employer un algorithme de réseau neural, un algorithme de correspondance de modèle et/ou un algorithme de corrélation pour analyser les signaux de capteurs pour détecter la présence de l'un ou plusieurs objets détectés et/ou de l'une ou plusieurs odeurs détectées.

3. Accessoire de véhicule (30, 40, 50, 60) selon la revendication 1, dans lequel la source d'odeurs (60) est utilisable pour fonctionner en conjonction avec un système de recirculation d'air ou un système de climatisation compris dans le véhicule (10) pour purger des odeurs d'une région intérieure du véhicule (10) qui ne sont plus requises par le processeur (50) et pour faire recirculer des odeurs requises à l'intérieur de la région intérieure du véhicule (10) pour réduire une consommation de matières de génération d'odeurs (200, 210) dans la source d'odeurs (60).

4. Accessoire de véhicule (30, 40, 50, 60) selon la revendication 1, dans lequel les capteurs (40) pour détecter des odeurs présentes dans l'environnement (20) sont mis en oeuvre sous la forme d'un réseau de capteurs de polymères conducteurs.

5. Accessoire de véhicule (30, 40, 50, 60) selon la revendication 4, dans lequel les capteurs (40) pour détecter des odeurs présentes dans l'environnement (20) sont capables d'être chauffés à une température élevée pour au moins partiellement expulser la contamination accumulée à l'intérieur de celui-ci.

6. Accessoire de véhicule (30, 40, 50, 60) selon la revendication 4, dans lequel les capteurs (40) pour détecter des odeurs présentes dans l'environnement (20) sont munis d'une ou plusieurs sources d'un gaz d'étalonnage à utiliser dans l'étalonnage périodique des capteurs (40).

7. Accessoire de véhicule (30, 40, 50, 60) selon la revendication 1, dans lequel la source d'odeurs (60) est munie d'une pluralité de matières qui sont susceptibles d'être vaporisées par excitation thermique et/ou ultrasonique pour générer des odeurs à dégager dans le véhicule (10).

8. Accessoire de véhicule (30, 40, 50, 60) selon la revendication 7, dans lequel la pluralité de matières susceptibles de générer des odeurs à dégager dans le véhicule (10) est fournie sous la forme d'une cartouche remplaçable (200).

9. Accessoire de véhicule (30, 40, 50, 60) selon la revendication 1, dans lequel les capteurs (30) pour détecter des objets visuels présents dans l'environnement (20) sont mis en oeuvre sous la forme d'une ou plusieurs caméras (30) dirigées dans au moins l'une de : une direction de déplacement vers l'avant du véhicule (10) et une direction latérale par rapport à la direction de déplacement du véhicule (10).

10. Accessoire de véhicule (30, 40, 50, 60) selon la revendication 9, dans lequel les capteurs (30) pour détecter des objets visuels présents dans l'environnement (20) sont montés à une ou plusieurs positions à l'intérieur du véhicule (10) auxquelles leur champ de vision est périodiquement soumis à une opération de nettoyage pour enlever des débris qui gênent la vision.
